(19) Europäisches Patentamt
European Patent Office
Office européen des brevets

(11) Veröffentlichungsnummer: **0 035 475**
**B1**

(12) # EUROPÄISCHE PATENTSCHRIFT

(45) Veröffentlichungstag der Patentschrift: **30.11.83**

(21) Anmeldenummer: **81810065.3**

(22) Anmeldetag: **27.02.81**

(51) Int. Cl.³: **C 07 D 263/14,** C 07 D 263/32, C 07 D 265/08, C 07 D 277/10, A 01 N 43/76, A 01 N 43/78, A 01 N 43/86

(54) **Herbizid wirkende 2-Nitro-5-phenoxyphenyl-oxazole, -oxazine, -imidazole, -pyrimidine und -thiazole, ihre Herstellung und Verwendung.**

(30) Priorität: **05.03.80 CH 1739/80**

(43) Veröffentlichungstag der Anmeldung: **09.09.81 Patentblatt 81/36**

(45) Bekanntmachung des Hinweises auf die Patenterteilung: **30.11.83 Patentblatt 83/48**

(84) Benannte Vertragsstaaten: **AT BE CH DE FR GB IT LI NL**

(56) Entgegenhaltungen:
**EP - A - 0 003 297**
**DE - A - 2 019 821**
**DE - A - 2 261 918**
**US - A - 3 928 416**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Patentabteilung Postfach, CH-4002 Basel (CH)**

(72) Erfinder: **Dürr, Dieter, Dr., Brändelistalweg 16, CH-4103 Bottmingen (CH)**

EP 0 035 475 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Herbizid wirkende 2-Nitro-5-phenoxyphenyl-oxazole, -oxazine, -imidazole, -pyrimidine und -thiazole, ihre Herstellung und Verwendung

Die vorliegende Erfindung betrifft neue 2-Nitro-5-phenoxy-phenyl-oxazole, -oxazoline, -oxazine, -imidazole, -pyrimidine und -thiazole, welche herbizide Wirkung haben, ferner Verfahren zu deren Herstellung, sie als Wirkstoff enthaltende Mittel sowie deren Verwendung zur selektiven Bekämpfung von Unkräutern in Kulturpflanzungen wie z. B. Getreide.

Herbizide Wirkung besitzende 2-Nitro-5-phenoxy-benzoesäurederivate sind beispielsweise aus der US-Patentschrift Nr. 3 928 416, Phenoxyalkyl-oxazoline aus der EP-A-0 003 297 bekanntgeworden. Die vorliegenden Verbindungen sind neu und zeichnen sich durch gute, gegenüber Getreiden selektive Herbizidwirkung aus, welche diejenige der bekannten Verbindungen übertrifft.

Die neuen 2-Nitro-5-phenoxyphenyl-oxazole, -oxazine, -imidazole, -pyrimidine und -thiazole entsprechen der Formel I

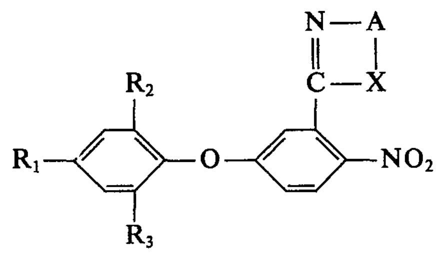

(I)

worin

A ein $C_2-C_3$ Alkylenrest oder $C_2-C_3$-Alkenylenrest, der durch $C_1-C_4$ Alkyl oder $C_1-C_4$ Haloalkyl substituiert sein kann,

$R_1$, $R_2$ und $R_3$ unabhängig voneinander je Wasserstoff, Halogen, Trifluormethyl, Nitro oder Cyan und

X Sauerstoff, Schwefel, die Iminogruppe $-NH-$ oder eine $C_1-C_4$ Alkyliminogruppe $\rangle$N-Alkyl bedeuten.

In diesen Definitionen können die Alkylreste geradkettig oder verzeigt sein. Unter Halogen wird Fluor, Chlor, Brom oder Jod verstanden, vorzugsweise Chlor oder Brom.

Die Verbindungen der Formel I werden auf verschiedenen, an sich bekannten Wegen hergestellt:

Gemäß einem ersten Verfahren werden die 2-Nitro-5-phenoxyphenyl-oxazole, -oxazine, -imidazole, -pyrimidine und -thiazole der Formel I hergestellt, indem man ein 2-Nitro-5-phenoxybenzoesäurehaloalkylamid oder -thiobenzoesäurehaloalkylamid der Formel II

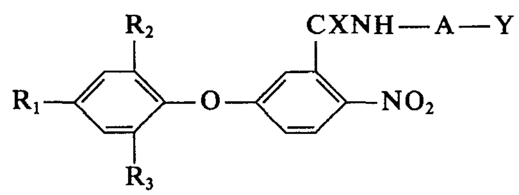

(II)

worin A, $R_1$, $R_2$, $R_3$ und X die unter Formel I gegebene Bedeutung haben, und Y für die OH-Gruppe, für ein Halogenatom oder für einen Sulfonsäureesterrest steht, in einem Lösungsmittel einem Ringschluß unterworfen werden.

Als Lösungsmittel kommen vor allem wasserlösliche in Frage, wie niedermolekulare Alkohole, Ketone, Dimethylformamid, Dimethylsulfoxyd aber auch wasserunlösliche chlorierte Kohlenwasserstoffe, Äther, und aromatische Kohlenwasserstoffe.

Der Ringschluß erfolgt in Gegenwart einer Base wie z. B. einem Alkalimetallhydroxyd oder einem quaternären Ammoniumhydroxyd, wenn Y ein Halogenatom oder einen Sulfonsäurerest bedeutet, jedoch unter sauren Bedingungen, z. B. in Gegenwart von Schwefelsäure, wenn Y die Hydroxylgruppe bedeutet.

Als Katalysator für den Ringschluß kommen die Alkalimetallsalze oder tertiären Ammoniumsalze von Halogeniden und Sulfonsäuren in Frage.

Der Ringschluß erfolgt bereits bei Raumtemperatur, zur Beschleunigung des Vorganges kann das Reaktionsgemisch bis zum Sieden erwärmt werden. Siehe dazu z. B. Synthesis 1980, p. 63.

Die Imidazol- und Pyrimidin-Verbindungen der Formel I werden dadurch hergestellt, daß man ein 1-Cyan-2-nitro-5-phenoxybenzol der Formel III

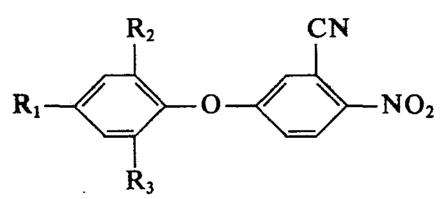

(III)

worin $R_1$, $R_2$ und $R_3$ die unter Formel I gegebene Bedeutung haben, in Gegenwart eines Überschusses eines Alkylendiamines der Formel IV,

$$R_4-NH-A-NH_2 \qquad (IV)$$

worin $R_4$ Wasserstoff oder $C_1-C_4$-Alkyl bedeutet und A die unter Formel I gegebene Bedeutung hat, in einem Druckgefäß, bei hoher Temperatur bis zum Ringschluß reagieren läßt.

Diese Methode ist z. B. auch in der DE-OS-2 830 141 beschrieben.

Die Ausgangsstoffe der Formel I können von bekannten 2-Nitro-5-phenoxybenzoesäuren ausgehend hergestellt werden.

Durch Behandeln dieser Säuren mit einem Halogenierungsmittel, wie Thionylchlorid, Thionylbromid, Phosphoroxychlorid oder -bromid, erhält man das entsprechende Säurehalogenid, an welches man dann ein Alkanolamin ankondensiert, gemäß dem Schema:

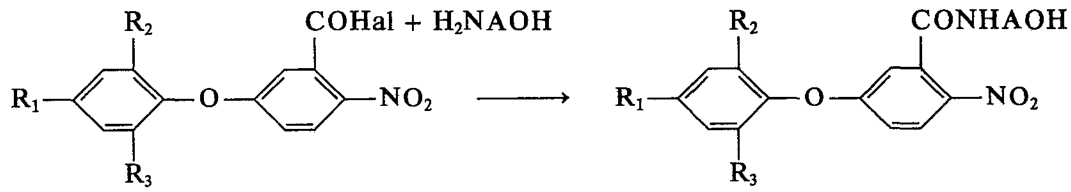

In diesen Formeln haben A, $R_1$, $R_2$ und $R_3$ die oben gegebene Bedeutung. Das entstandene 2-Nitro-5-phenoxy-benzoesäurealkanolamid wird ebenfalls mit einem Halogenierungsmittel behandelt, wobei sich die obengenannten gut eignen, und zum Haloalkylamid mit der Formel II umgesetzt.

Die Ausgangsstoffe der Formel III sind bekannt (siehe US-P-3 928 416), oder können in an sich bekannter Weise durch Kondensieren von entsprechend substituiertem Halobenzol und Phenol in Gegenwart eines säurebindenden Mittels hergestellt werden.

Als Alkyldiamine der Formel IV kommen vor allem die bekannten Äthylendiamin, 1,2- und 1,3-Propylendiamin, 1,2-, 2,3- sowie 1,3-Butylendiamine in Betracht.

Die Verbindungen der Formel I haben gute herbizide Eigenschaften. In großen Aufwandmengen können sie als Totalherbizide eingesetzt werden. Interessanter jedoch ist ihr Einsatz in Aufwandmengen von ca. 0,1 bis 5 kg pro Hektar als Selektivherbizide in Kulturpflanzungen, bei gekeimten im Nachauflaufverfahren oder bei frisch gesäten im Vorauflaufverfahren. Sie hemmen oder beeinträchtigen dort das Aufkommen von Unkräutern, zweikeimblättrigen vor allem aber auch von vielen einkeimblättrigen Arten wie Lolium, Alopecurus, Rottboellia, Sorghum, Digitaria, Setaria und Panicum. Kulturpflanzen, wie Getreide, Gerste, Weizen, Roggen, Mais oder Reis aber auch andere wie Baumwolle und Soja werden dabei ganz oder mindestens bei der Aufwandmenge von 1 kg/ha geschont.

Einige der neuen Wirkstoffe eignen sich auch für die Desiccation und Defoliation von Baumwoll- oder Kartoffelkulturen, kurz bevor deren Ernte.

Übliche Aufwandmengen an erfindungsgemäßen Herbizid pro Hektar schwanken je nach Aktivität des eingesetzten Wirkstoffs, Bodenbeschaffenheit, Klima- und Witterungsbedingungen, Art und Zeitpunkt der Anwendung und Art der Kultur und der zu bekämpfenden Unkräuter zwischen 0,1 und 10,0 kg und liegen vorzugsweise zwischen 0,5 und 4,0 kg.

Ferner besitzen die Verbindungen der Formel I günstige wachstumsregulierende Effekte (Wuchshemmung). Insbesondere hemmen sie das Wachstum der Pflanzen. Beispiele für die nutzbringende Anwendung der erfindungsgemäßen Verbindungen sind z. B.

- die Reduktion des vegetativen Wachstums bei Soja und ähnlichen Leguminosen, was zu einer Ertragssteigerung dieser Kultur führt;
- die Hemmung des Wachstums von Gras, zwecks Einsparung an Schnittarbeit;
- die Hemmung des vegetativen Wachstums bei Getreiden, was zu Pflanzen mit kürzeren, festen Stengeln führt, die sich durch Einwirkung von Wind und Regen nicht so leicht umlegen lassen.

Die Verbindungen der Formel I wirken alle gut. Die besten Resultate wurden mit den Verbindungen erreicht, in denen

X Sauerstoff ist, speziell mit denjenigen in denen
$R_1$ Wasserstoff oder Halogen,
$R_2$ Trifluormethyl oder Halogen,
$R_3$ Wasserstoff,
A eine Äthylen-, Vinylen- oder Propylengruppe, die gegebenenfalls durch Methyl oder Halomethyl substituiert sein kann, und
X Sauerstoff bedeuten.

Die neuen Wirkstoffe der Formel I sind stabile Verbindungen, welche in üblichen organischen Lösungsmitteln, wie Alkoholen, Äthern, Ketonen, Dimethylformamid oder Dimethylsulfoxid löslich sind. Sie sind nicht explosiv oder ätzend, und ihre Handhabung bedarf keiner besonderer Vorsichtsmaßnahmen.

Die Herstellung erfindungsgemäßer Mittel erfolgt in an sich bekannter Weise durch inniges Vermischen und Vermahlen von Wirkstoffen der Formel I mit geeigneten Trägerstoffen und/oder Verteilungsmitteln, gegebenenfalls unter Zusatz von gegenüber den Wirkstoffen inerten Antischaum-, Netz-, Dispersions- und/oder Lösungsmitteln. Die Wirkstoffe können in den folgenden Aufarbeitungsformen vorliegen und angewendet werden:

Feste Aufarbeitungsformen:
  Stäubemittel, Streumittel, Granulate, Umhüllungsgranulate, Imprägnierungsgranulate und Homogengranulate;
In Wasser dispergierbare Wirkstoffkonzentrate:
  Spritzpulver (wettable powder), Pasten, Emulsionen, Emulsionspräparate;
Flüssige Aufarbeitungsformen:
  Lösungen, Dispersionen.

Die Wirkstoffkonzentrationen betragen in den erfindungsgemäßen Mitteln 1 bis 80 Gewichtsprozent und können gegebenenfalls bei der Anwendung auch in geringen Konzentrationen wie etwa 0,05 bis 1% vorliegen.

Den beschriebenen erfindungsgemäßen Mitteln lassen sich andere biozide Wirkstoffe oder Mittel beimischen. So können die neuen Mittel außer den genannten Verbindungen der Formel I z. B. Insektizide, Fungizide, Bakterizide, Fungistatika, Bakteriostatika, Nematozide oder weitere Herbizide zur Verbreiterung des Wirkungsspektrums enthalten.

Im nachfolgenden Beispiel wird die Herstellung eines 2-(2'-Nitro-5'-phenoxyphenyl)-2-oxazolins der Formel I veranschaulicht. Weitere in entsprechender Weise hergestellte Wirkstoffe sind in der anschließenden Tabelle aufgeführt. Temperaturen sind in Celsius-Graden angegeben, Druckangaben in Millibar (mbar). Teile und Prozentangaben beziehen sich auf das Gewicht. In weiteren Beispielen wird die Aufarbeitung der Wirkstoffe zu technisch anwendbaren Verabreichungsformen und die herbizide, und pflanzenwuchshemmende Wirkung der neuen Verbindungen gezeigt.

## Beispiel 1

2-[2'-Nitro-5'-(2''-chlor-4''-trifluormethylphenoxy)-phenyl]-2-oxazolin

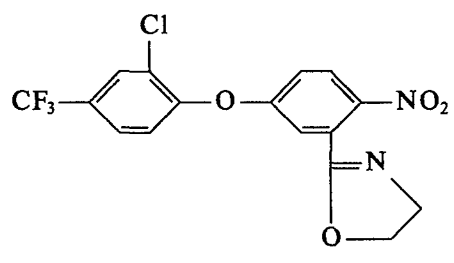

a) Eine Lösung von 184 g (0,5 Mol) 2-Nitro-5-(2'-chlor-4'-trifluormethylphenoxy)-benzoylchlorid in 500 ml Toluol wird bei 20 – 40° C zu einer Lösung von 90 g Äthanolamin in 300 ml Dioxan getropft. Nach 3 Stunden wird durch Zugabe von Eiswasser das entstandene 2-Nitro-5-(2'-chlor-4'-trifluormethylphenoxy)-benzoesäure-$\beta$-hydroxyäthylamid ausgefällt. Nach dem Trocknen erhält man 199 g (98% der Theorie) Material vom Schmelzpunkt 142 – 143° C.

b) 199 g dieses Hydroxyäthylamides werden mit 150 g Thionylchlorid und 400 ml Toluol verrührt und dann 20 Stunden bei 40° C gehalten. Dann dampft man die Reaktionslösung am Vakuum ein. Der Rückstand wird in 500 ml Methylenchlorid und mit 0,6 g Tetrabutylammoniumchlorid verrührt und dann 42 g gepulvertem Natriumhydroxyd versetzt. Man erwärmt 30 Minuten an Rückfluß und rührt während weiteren 10 Stunden. Zur Aufarbeitung wird das Gemisch in Wasser gegossen, die organische Phase abgetrennt, gewaschen, getrocknet und zur Trockne eingedampft. Als Rückstand verbleibt die Titelverbindung als klares, zähes Öl mit dem Brechungsindex $n_D^{25}$ = 1.5703.

Ausbeute 109 g (79% der Theorie).

Analyse:

| | | |
|---|---|---|
| $C_{ber.}$ 49,70% | $H_{ber.}$ 2,61% | $N_{ber.}$ 7,24% |
| $C_{ber.}$ 49,5% | $H_{gef.}$ 2,6% | $N_{gef.}$ 7,1% |

Molekulargewicht 346.71. Das NMR-Spektrum steht mit der Struktur im Einklang.
In analoger Weise zu diesem Beispiel werden folgende Verbindungen hergestellt:

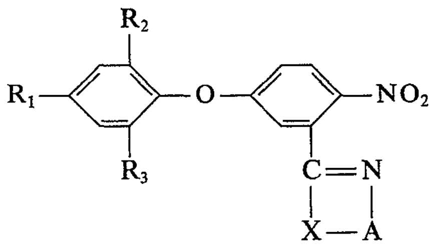

| No. | $R_1$ | $R_2$ | $R_3$ | 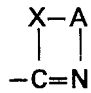 | Phys. Daten |
|---|---|---|---|---|---|
| 1 | $CF_3$ | Cl | H | 2-oxazolin-2-yl | $n_D^{25}$ 1.5703 |
| 2 | $CF_3$ | Cl | H | 5-methyl-2-oxazolin-2-yl | $n_D^{25}$ 1.5495 |
| 3 | $CF_3$ | Cl | H | 5,6-dihydro-4 H-1,3-oxazin-2-yl | Smp. 84° |
| 4 | $CF_3$ | Cl | H | 4,4-dimethyl-2-oxazolin-2-yl | $n_D^{27}$ 1.5458 |
| 5 | $CF_3$ | Cl | H | 4-methyl-4-chlormethyl-2-oxazolin-2-yl | $n_D^{27}$ 1.5490 |
| 6 | $CF_3$ | Cl | H | 5-methyl-oxazol-2-yl | $n_D^{26}$ 1.5725 |
| 7 | $CF_3$ | Cl | H | 4-äthyl-2-oxazolin-2-yl | $n_D^{22}$ 1.5505 |
| 8 | $CF_3$ | Cl | H | 4-methyl-2-oxazolin-2-yl | Smp. 65—67° |
| 9 | $CF_3$ | Cl | Cl | 2-oxazolin-2-yl | Smp. 118—122° |
| 10 | Cl | Cl | H | 2-oxazolin-2-yl | |
| 11 | Cl | Cl | H | 4,4-dimethyl-2-oxazolin-2-yl | |
| 12 | Br | Br | H | 2-oxazolin-2-yl | |
| 13 | $CF_3$ | CN | H | 2-oxazolin-2-yl | |
| 14 | Cl | CN | H | 5,6-dihydro-4 H-1,3-oxazin-2-yl | |
| 15 | $CF_3$ | $NO_2$ | H | 2-oxazolin-2-yl | |
| 16 | Cl | $NO_2$ | H | 2-oxazolin-2-yl | |
| 17 | Cl | Cl | Cl | 2-oxazolin-2-yl | |
| 18 | Cl | $CF_3$ | H | 2-oxazolin-2-yl | |
| 19 | $CF_3$ | Cl | H | 2-thiazolin-2-yl | Smp. 140° |
| 20 | $CF_3$ | Cl | H | thiazol-2-yl | |
| 21 | $CF_3$ | Cl | Cl | 2-thiazolin-2-yl | |
| 22 | Cl | Cl | H | 2-thiazolin-2-yl | |
| 23 | Cl | Cl | H | thiazol-2-yl | |
| 24 | Cl | Cl | Cl | 2-thiazolin-2-yl | |
| 25 | $CF_3$ | Cl | H | 5,6-dihydro-4 H-pyrimidin-2-yl | |
| 26 | $CF_3$ | Cl | H | 1-methyl-2-imidazolin-2-yl | |
| 27 | $CF_3$ | Cl | H | imidazol-2-yl | |

Beispiel 2

Die Aufbereitung der Wirkstoff-Verbindungen der Formel I zu für die Landwirtschaft brauchbaren Anwendungsformen kann beispielsweise gemäß einer der folgenden Vorschriften erfolgen:

## Granulat

Zur Herstellung eines 5%igen Granulates werden die folgenden Stoffe verwendet:

5 Teile 2-[2'-Nitro-5-(2''-chlor-4''-trifluormethylphenoxy)phenyl]-2-oxazolin
0,25 Teile epoxidiertes Pflanzenöl
0,25 Teile Cetylpolyglykoläther,
3,50 Teile Polyäthylenglykol,
91 Teile Kaolin (Korngröße: 0,3 – 0,8 mm).

Die Aktivsubstanz wird mit dem Pflanzenöl vermischt und in 6 Teilen Aceton gelöst, hierauf wird Polyäthylenglykol und Cetylpolyglykoläther zugesetzt. Die so erhaltene Lösung wird auf Kaolin aufgesprüht und anschließend im Vakuum verdampft.

## Spritzpulver

Zur Herstellung eines a) 70%igen und b) 10%igen Spritzpulvers werden folgende Bestandteile verwendet:

a) 70 Teile 2-[2'-Nitro-5-(2''-chlor-4''-trifluormethylphenoxy)-phenyl]-2-oxazolin
5 Teile Natriumdibutylnaphthylsulfat,
3 Teile Naphthalinsulfonsäure-Phenolsulfonsäuren-Formaldehyd-Kondensat 3 : 2 : 1,
10 Teile Kaolin,
12 Teile Champagne-Kreide;
b) 10 Teile des obigen Wirkstoffes
3 Teile Gemisch der Natriumsalze von gesättigten Fettalkoholsulfaten,
5 Teile Naphthalinsulfonsäuren-Formaldehyd-Kondensat,
82 Teile Kaolin.

Der angegebene Wirkstoff wird auf die entsprechenden Trägerstoffe (Kaolin und Kreide) aufgezogen und anschließend mit den übrigen Bestandteilen vermischt und vermahlen. Man erhält Spritzpulver von vorzüglicher Benetzbarkeit und Schwebefähigkeit. Aus solchen Spritzpulvern können durch Verdünnen mit Wasser Suspensionen von 0,1 – 8% Wirkstoff erhalten werden, die sich zur Unkrautbekämpfung in Pflanzenkulturen eignen.

## Emulsionskonzentrat

Zur Herstellung eines 25%igen Emulsionskonzentrates werden

25 Teile 2-[2'-Nitro-5-(2''-chlor-4''-trifluormethylphenoxy)-phenyl]-2-oxazolin
5 Teile einer einer Mischung von Nonylphenolpolyoxyäthylen und Calciumdodecylbenzolsulfat,
15 Teile Cyclohexanon,
55 Teile Xylol

miteinander vermischt. Dieses Konzentrat kann mit Wasser zu Emulsionen auf geeignete Konzentrationen von z. B. 0,1 bis 10% verdünnt werden. Solche Emulsionen eignen sich zur Bekämpfung von Unkräutern in Kulturpflanzen.

## Paste

Zur Herstellung einer 45%igen Paste werden folgende Stoffe verwendet:

a) 45 Teile 2-[2'-Nitro-5-(2''-chlor-4''-trifluormethylphenoxy)-phenyl]-2-oxazolin
5 Teile Natriumaluminiumsilikat,
14 Teile Cetylpolyglykoläther mit 8 Mol Äthylenoxid,
1 Teil Oleylpolyglykoläther mit 5 Mol Äthylenoxid,
2 Teile Spindelöl,
10 Teile Polyäthylenglykol,
23 Teile Wasser,
b) 45 Teile 2-[2'-Nitro-5-(2''-chlor-4''-trifluormethylphenoxy)-phenyl]-2-oxazolin
5 Teile Äthylenglycol
3 Teile Octylphenoxypolyäthylenglycol mit 9 – 10 Mol Äthylenoxyd pro Mol Octylphenol

| | |
|---|---|
| 3 Teile | von einem Gemisch aromatischer Sulfonsulfonsäuren, kondensiert mit Formaldehyd als Ammoniumsalz |
| 1 Teil | Siliconöl in Form einer 75%igen Emulsion |
| 0,1 Teil | einer Mischung von 1-(3-Chlorallyl) 3,5,7-triazo-azonium-adamantan-chlorid mit Natriumcarbonat, Chloridwert mind. 11,5% |
| 0,2 Teile | eines biopolymeren Verdickers mit max. 100 Keimen pro Gramm |
| 42,7 Teile | Wasser |

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

## Beispiel 3

Die herbizide und pflanzenwuchshemmende Wirkung der Verbindungen der Formel I wurde durch folgende Versuche ermittelt:

### Pre-emergente Herbizidwirkung (Vorauflaufwirkung)

Im Gewächshaus werden Pflanzensamen in Blumentöpfe von 12—15 cm Durchmesser gesät, so daß sich pro Topf 10—25 Pflänzchen entwickeln können. Unmittelbar nach der Einsaat wird die Erdoberfläche mit einer wäßrigen Suspension der Wirkstoffe, erhalten aus einem 10%igen Spritzpulver, behandelt. Es werden zwei verschiedene Konzentrationsreihen angewendet, entsprechend 2 und 1 kg Wirksubstanz pro Hektar. Die Blumentöpfe werden im Gewächshaus bei 22—25°C und 50—70% relativer Luftfeuchtigkeit und regelmäßigem Begießen gehalten und der Versuch nach 3 Wochen ausgewertet. Der Zustand der Pflanzen wurde gemäß dem folgenden EWRC-(European Weed Research Council)-Bewertungsschema beurteilt.

| | |
|---|---|
| 9 | Pflanze gedeiht wie unbehandelte Kontrolle |
| 6—9 | leichte Schäden |
| 5 | mittlere Schäden |
| 2—4 | schwere Schäden |
| 1 | Pflanze abgestorben. |

Die Resultate für die Verbindungen 1—4 sind wie folgt:

| | Verbindung 1 | | 2 | | 3 | | 4 | |
|---|---|---|---|---|---|---|---|---|
| | Aufwandmenge kg/ha | | | | | | | |
| | 2 | 1 | 2 | 1 | 2 | 1 | 2 | 1 |
| Pflanze | | | | | | | | |
| Gerste | 8 | 8 | 8 | 9 | 6 | 9 | 9 | 9 |
| Weizen | 8 | 8 | 8 | 9 | 6 | 8 | 8 | 9 |
| Mais | 6 | 8 | 9 | 9 | 8 | 9 | 7 | 9 |
| Reis | 6 | 7 | 7 | 9 | 6 | 8 | 6 | 9 |
| alopecurus myosuroides | 1 | 2 | 1 | 2 | 1 | 2 | 1 | 1 |
| digitaria sanguinalis | 1 | 1 | 1 | 1 | 1 | 1 | 1 | 1 |
| echinochloa crus galli | 1 | 1 | 1 | 2 | 1 | 3 | 1 | 1 |
| sorghum halepense | 2 | 2 | 1 | 1 | 1 | 1 | 2 | 3 |
| rottboellia exaltata | 1 | 1 | 1 | 2 | 1 | 1 | 1 | 3 |
| abutilon sp. | 1 | 1 | 1 | 2 | 1 | 1 | 1 | 2 |
| sida spinosa | 1 | 1 | 1 | 1 | 1 | 1 | 1 | 2 |
| amarantus rehoflexus | 1 | 1 | 1 | 1 | 1 | 1 | 1 | 1 |
| sinapis alba | 1 | 1 | 1 | 2 | 1 | 4 | 2 | 3 |
| solanum nigrum | 1 | 1 | 1 | 1 | 1 | 1 | 1 | 1 |
| stellaria media | 1 | 1 | 1 | 1 | 1 | 1 | 1 | 3 |
| chrysanthemum leuc. | 1 | 1 | 1 | 1 | 1 | 1 | 1 | 1 |
| galium aparine | 1 | 1 | 2 | 4 | 2 | 4 | 1 | 1 |
| viola tricolor | 1 | 1 | 1 | 1 | 1 | 1 | 1 | 1 |
| veronica sp. | 1 | 1 | 1 | 1 | 1 | 1 | 1 | 1 |

Herbizide Wirkung bei Applikation der Wirkstoffe nach dem Auflaufen der Pflanzen (post-emergent)

Verschiedene Kulturpflanzen und Unkräuter werden aus den Samen in Töpfen im Gewächshaus aufgezogen, bis sie das 4- bis 6-Blatt-Stadium erreicht haben. Dann werden die Pflanzen mit wässerigen Wirkstoffemulsionen (erhalten aus dem 25%igen Emulsionskonzentrat) in Dosierungen von 2 und 1 kg/ha gespritzt. Die behandelten Pflanzen werden dann bei optimalen Bedingungen von Licht, regelmäßigem Begießen, 22–25°C Temperatur und 50–70%iger relativer Luftfeuchtigkeit gehalten. Die Auswertung des Versuches erfolgt 15 Tage nach der Behandlung nach dem oben gegebenen Bewertungsschema. Die Resultate für die Verbindungen 2 und 4 sind wie folgt:

| | Verbindung No. 2 | | 4 | |
|---|---|---|---|---|
| | Aufwandmenge kg/ha | | | |
| | 2 | 1 | 2 | 1 |
| Pflanze | | | | |
| Weizen | 7 | 9 | 6 | 6 |
| alopecurus myosuroides | 3 | 4 | 3 | 3 |
| echinochloa crus galli | 2 | 3 | 1 | 1 |
| abutilon sp. | 1 | 1 | 1 | 1 |
| sida spinosa | 2 | 2 | 3 | 3 |
| amarantus retroflexus | 1 | 1 | 1 | 1 |
| chenopodium album | 1 | 1 | 1 | 1 |
| solanum nigrum | 1 | 1 | 1 | 1 |
| ipomoea purpurea | 1 | 1 | 1 | 1 |
| sinapis alba | 1 | 1 | 1 | 1 |
| stellaria media | 1 | 1 | 1 | 1 |
| chrysanthemum leucum | 1 | 1 | 1 | 1 |
| galium aparine | 1 | 1 | 1 | 1 |
| viola tricolor | 1 | 1 | 1 | 1 |
| veronica sp. | 1 | 1 | 1 | 1 |

Selektive herbizide Wirkung auf Reis im Nachauflaufverfahren

Reispflänzchen, welche 25 Tage alt sind, wurden im Gewächshaus in große rechteckige Eternitschalen verpflanzt. Zwischen die Reihen der Reispflanzen wurden dann Samen der in Reiskulturen vorkommenden Unkräuter Echinochloa crus galli, Cyperus difformis, Ammania und Rotala gesät. Die Schalen wurden gut bewässert und bei einer Temperatur von ca. 25°C und hoher Luftfeuchtigkeit gehalten. Nach 12 Tagen, wenn die Unkräuter aufgelaufen sind und das 2–3 Blattstadium erreicht haben, wurde die Erde in der Schale mit einer 2,5 cm hohen Schicht Wasser bedeckt. Der Wirkstoff wurde dann als Emulsionskonzentrat mittels einer Pipette zwischen die Pflanzenreihen appliziert, wobei man das Emulsionskonzentrat so verdünnte und auftrug, daß es einer Applikationsmenge im Feld von 1 und 1/2 kg Wirkstoff pro Hektar entsprach. Der Versuch wurde nach 4 Wochen ausgewertet. Die Resultate für die Verbindungen 1 und 2 sind wie folgt:

| | Verbindung No. 1 | | 2 | |
|---|---|---|---|---|
| | Aufwendmenge kg/ha | | | |
| | 1 | 1/2 | 1 | 1/2 |
| Reis | 6 | 7 | 6 | 6 |
| echinochloa curs galli | 2 | 3 | 1 | 2 |
| cyperus difformis | 4 | 4 | 1 | 2 |
| ammania indica | 1 | 1 | 1 | 1 |
| rotala indica | 1 | 4 | 2 | 3 |

Desiccations- und Defoliationswirkung

In einem Gewächshaus wurden Baumwollpflanzen der Sorte Deltapine in Tontöpfen angezogen. Nach abgeschlossener Kapselbildung wurden sie mit wässerigen Zubereitungen des Wirkstoffes Nr. 1 in einer Aufwandmenge, die 1,2, 0,6 und 0,3 kg/ha im Feld entspräche, gespritzt. Unbehandelte Pflanzen wurden als Kontrolle belassen. Die Auswertung des Versuches erfolgte 3, 7 und 14 Tage nach Applikation der Wirksubstanz durch Bestimmen des Grades an Defoliation (% abgefallene Blätter) und an Desiccation (% Austrocknung der an der Pflanze verbleibenden Blätter).

In diesem Versuch beließen die Verbindungen Nr. 1 bis 7 bei Aufwandmengen von 0,6 und 1,2 kg/ha nach 7 Tagen bloß noch wenige vertrocknete Blätter auf den Pflanzen (>80% Blattfall und Austrocknung). Die beste Wirkung zeigte die Verbindung Nr. 3.

**Patentansprüche**

1. 2-Nitro-5-phenoxyphenyl-oxazole, -oxazine, -imidazole, -pyrimidine oder -thiazole der Formel I

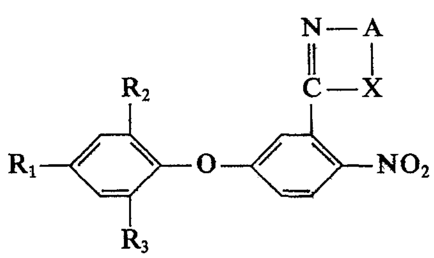

(I)

worin
A einen $C_2-C_3$-Alkylenrest oder $C_2-C_3$-Alkenylenrest, der durch $C_1-C_4$ Alkyl oder $C_1-C_4$-Haloalkyl substituiert sein kann,
$R_1$, $R_2$ und $R_3$ unabhängig voneinander je Wasserstoff, Halogen, Trifluormethyl, Nitro oder Cyan,
X Sauerstoff, Schwefel, die Iminogruppe $-NH-$ oder eine $C_1-C_4$-Alkyliminogruppe $>$N-Alkyl bedeuten.

2. 2-Nitro-5-phenoxyphenyl-oxazole und -oxazine gemäß Formel I, Anspruch 1, worin X Sauerstoff ist, während A, $R_1$, $R_2$ und $R_3$ die im Anspruch 1 gegebene Bedeutung haben.

3. 2-Nitro-5-phenoxyphenyl-thiazole gemäß Formel I, Anspruch 1, worin X Schwefel ist, während A, $R_1$, $R_2$ und $R_3$ die im Anspruch 1 gegebene Bedeutung haben.

4. 2-Nitro-5-phenoxyphenyl-oxazole, -oxazine und -thiazole der Formel I, Anspruch 1, worin

$R_1$ Wasserstoff oder Halogen,
$R_2$ Trifluormethyl oder Halogen,
$R_3$ Wasserstoff oder Chlor,
A ein Äthylen-, Vinylen- oder Propylenrest, der durch Methyl oder Halomethyl substituiert sein kann und
X Sauerstoff bedeutet.

5. 2-[2'-Nitro-5'-(2''-chlor-4''-trifluormethylphenoxy)-phenyl]-2-oxazolin.

6. 2-[2'-Nitro-5'-(2''-chlor-4''-trifluormethylphenoxy)-phenyl]-5-methyl-2-oxazolin.

7. 2-[2'-Nitro-5'-(2''-chlor-4''-trifluormethylphenoxy)-phenyl]-5,6-dihydro-4H-1,3-oxazin.
8. 2-[2'-Nitro-5'-(2''-chlor-4''-trifluormethylphenoxy)-phenyl]-4,4-dimethyl-2-oxazolin.
9. 2-[2'-Nitro-5'-(2''-chlor-4''-trifluormethylphenoxy)-phenyl]-4-methyl-4-chlormethyl-2-oxazolin.
10. 2-[2'-Nitro-5-(2''-chlor-4''-trifluormethylphenoxy)-phenyl]-5-methyl-oxazol.
11. 2-[2'-Nitro-5'-(2''-chlor-4''-trifluormethylphenoxy)-phenyl]-4-methyl-2-oxazolin.
12. 2-[2'-Nitro-5'-(2''-chlor-4''-trifluormethylphenoxy)-phenyl]-4-äthyl-2-oxazolin.
13. 2-[2'-Nitro-5-(2'',6''-dichlor-4''-trifluormethylphenoxy)-phenyl]-2-oxazolin.
14. 2-[2'-Nitro-5-(2''-chlor-4''-trifluormethylphenoxy)-phenyl]-2-thiazolin-2-yl.
15. Verfahren zur Herstellung der Oxazol-, Oxazin-, Imidazol-, Pyrimidin- und Thiazolverbindungen der Formel I gemäß Anspruch 1, dadurch gekennzeichnet, daß man ein 2-Nitro-5-phenoxy-benzoesäurehalogenid der Formel II

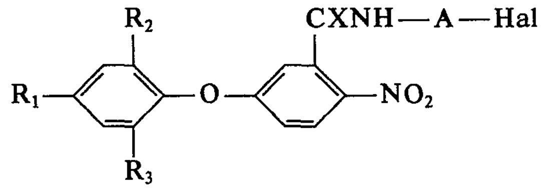

(II)

worin A, $R_1$, $R_2$, $R_3$ und X die in Anspruch 1 gegebene Bedeutung haben, in einem inerten Lösungs- und Verdünnungsmittel mit einem Alkalimetallhydroxyd behandelt.
16. Herbizides und den Pflanzenwuchs hemmendes Mittel, dadurch gekennzeichnet, daß es als wirksame Komponente mindestens eine Verbindung der Formel I gemäß Anspruch 1 enthält.
17. Verwendung der Verbindungen der Formel I gemäß Anspruch 1 zur Bekämpfung unerwünschten Pflanzenwachstums.
18. Verwendung der Verbindungen der Formel I gemäß Anspruch 1 zur selektiven Bekämpfung von Unkräutern in Nutzpflanzenkulturen.
19. Verwendung der Verbindungen der Formel I gemäß Anspruch 1 zur selektiven Bekämpfung von Unkräutern in Kulturen von Getreiden, Reis und Mais.
20. Verwendung der Verbindungen der Formel I gemäß Anspruch 1 zum Entblättern und Vertrocknen oberirdischer Pflanzenteile.

**Claims**

1. A 2-nitro-5-phenoxyphenyloxazole, -oxazine, -imidazole, -pyrimidine or -thiazole of the formula I

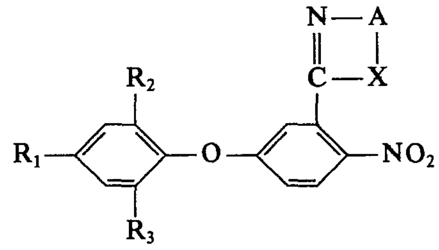

(I)

wherein A is a $C_2-C_3$alkylene or $C_2-C_3$ alkenylene radical which can be substituted by $C_1-C_4$alkyl or $C_1-C_4$haloalkyl, each of $R_1$, $R_2$ and $R_3$ independently is hydrogen, halogen, trifluoromethyl, nitro or cyano, and X is oxygen, sulfur, the imino group $-NH-$ or a $C_1-C_4$alkylimino group $>$N-alkyl.
2. A 2-nitro-5-phenoxyphenyloxazole or -oxazine of the formula I according to claim 1, wherein X is oxygen, and A, $R_1$, $R_2$ and $R_3$ are as defined in claim 1.
3. A 2-nitro-5-phenoxyphenylthiazole of the formula I according to claim 1, wherein X is sulfur, and A, $R_1$, $R_2$ and $R_3$ are as defined in claim 1.
4. A 2-nitro-5-phenoxyphenyloxazole, -oxazine or -thiazole of the formula I according to claim 1, wherein $R_1$ is hydrogen or halogen, $R_2$ is trifluoromethyl or halogen, $R_3$ is hydrogen or chlorine, A is an ethylene, vinylene or propylene radical which can be substituted by methyl or halomethyl, and X is oxygen.
5. 2-[2'-Nitro-5'-(2''-chloro-4''-trifluoromethylphenoxy)phenyl]-2-oxazoline.
6. 2-[2'-Nitro-5'-(2''-chloro-4''-trifluoromethylphenoxy)phenyl]-5-methyl-2-oxazoline.
7. 2-[2'-Nitro-5'-(2''-chloro-4''-trifluoromethylphenoxy)phenyl]-5,6-dihydro-4H-1,3-oxazine.
8. 2-[2'-Nitro-5'-(2''-chloro-4''-trifluoromethylphenoxy)phenyl]-4,4-dimethyl-2-oxazoline.
9. 2-[2'-Nitro-5'-(2''-chloro-4''-trifluoromethylphenoxy)phenyl]-4-methyl-4-chloromethyl-2-oxazoline.
10. 2-[2'-Nitro-5-(2''-chloro-4''-trifluoromethylphenoxy)phenyl]-5-methyl-oxazole.
11. 2-[2'-Nitro-5'-(2''-chloro-4''-trifluoromethylphenoxy)phenyl]-4-methyl-2-oxazoline.

12. 2-[2'-Nitro-5'-(2''-chloro-4''-trifluoromethylphenoxy)phenyl]-4-ethyl-2-oxazoline.
13. 2-[2'-Nitro-5-(2'',6''-dichloro-4''-trifluoromethylphenoxy)phenyl]-2-oxazoline.
14. 2-[2'-Nitro-5-(2''-chloro-4''-trifluoromethylphenoxy)phenyl]-2-thiazolin-2-yl.
15. A process for the production of an oxazole, oxazine, imidazole, pyrimidine or thiazole compound of the formula I according to claim 1, which process comprises treating a 2-nitro-5-phenoxybenzoylhalide of the formula II

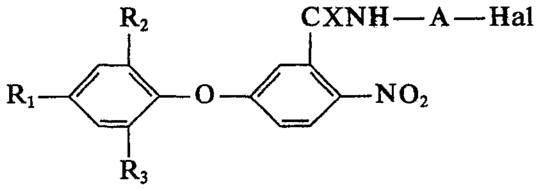

(II)

wherein A, $R_1$, $R_2$, $R_3$ and X are as defined in claim 1, with an alkali metal hydroxide, in an inert solvent or diluent.
16. A herbicidal and plant growth-inhibiting composition which contains, as active component, at least one compound of the formula I according to claim 1.
17. A method of controlling undesired plant growth at a locus, which comprises applying to said locus a herbicidally effective amount of a compound of the formula I according to claim 1.
18. A method of selectively controlling weeds in crops of useful plants, which comprises applying thereto a herbicidally effective amount of a compound of the formula I according to claim 1.
19. A method of selectively controlling weeds in crops of cereals, rice and maize, which comprises applying thereto a herbicidally effective amount of a compound of the formula I according to claim 1.
20. A method of defoliating and desiccating parts of plants above the soil, which comprises applying thereto a herbicidally effective amount of a compound of the formula I according to claim 1.

**Revendications**

1. 2-nitro-5-phénoxyphényl-oxazoles, -oxazines, -imidazoles, -pyrimidines ou -thiazoles de formule I

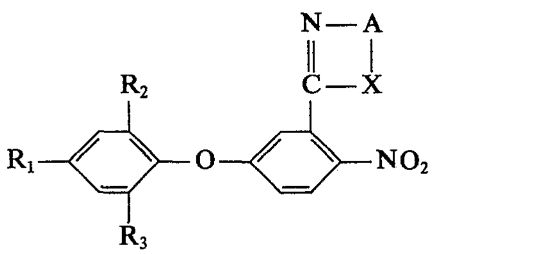

(I)

où A représente un radical alcoylène en $C_2$ à $C_3$ ou un radical alcénylène en $C_2$ à $C_3$, qui peut être substitué par un alcoyle en $C_1$ à $C_4$ ou un haloalcoyle en $C_1$ à $C_4$,
$R_1$, $R_2$ et $R_3$ représentent chacun indépendamment l'un de l'autre un hydrogène, un halogène, un trifluorométhyle, un nitro ou un cyano,
X représente un oxygène, un soufre, le groupe imino $-NH-$ ou un groupe alcoyle en $C_1$ à $C_4$-imino $>N$-alcoyle.
2. 2-nitro-5-phénoxyphényl-oxazoles et -oxazines selon la formule I de la revendication 1, où X représente un oxygène tandis que A, $R_1$, $R_2$ et $R_3$ ont la signification donnée dans la revendication 1.
3. 2-nitro-5-phénoxyphényl-thiazoles selon la formule I de la revendication 1, où X est un soufre, tandis que A, $R_1$, $R_2$ et $R_3$ ont la signification donnée dans la revendication 1.
4. 2-nitro-5-phénoxyphényl-oxazoles, -oxazines et -thiazoles de formule I de la revendication 1, où $R_1$ représente un hydrogène ou un halogène,
$R_2$ représente un trifluorométhyle ou un halogène,
$R_3$ représente un hydrogène ou un chlore,
A représente un radical acétylène, vinylène ou propylène, qui peut être substitué par un méthyle ou un halométhyle, et
X représente un oxygène.
5. 2-[2'-nitro-5'-(2''-chloro-4''-trifluorométhylphénoxy)-phényl]-2-oxazoline.
6. 2-[2'-nitro-5'-(2''-chloro-4''-trifluorométhylphénoxy)-phényl]-5-méthyl-2-oxazoline.
7. 2-[2'-nitro-5'-(2''-chloro-4''-trifluorométhylphénoxy)-phényl]-5,6-dihydro-4H-1,3-oxazine.
8. 2-[2'-nitro-5'-(2''-chloro-4''-trifluorométhylphénoxy)-phényl]-4,4-diméthyl-2-oxazoline.
9. 2-[2'-nitro-5'-(2''-chloro-4''-trifluorométhylphénoxy)-phényl]-4-méthyl-4-chlorométhyl-2-oxazoline.

10. 2-[2'-nitro-5-(2''-chloro-4''-trifluorométhylphénoxy)-phényl]-5-méthyl-oxazole.

11. 2-[2'-nitro-5'-(2''-chloro-4''-trifluorométhylphénoxy)-phényl]-4-méthyl-2-oxazoline.

12. 2-[2'-nitro-5'-(2''-chloro-4''-trifluorométhylphénoxy)-phényl]-4-éthyl-2-oxazoline.

13. 2-[2'-nitro-5-(2'',6''-dichloro-4''-trifluorométhylphénoxy)-phényl]-2-oxazoline.

14. 2-[2'-nitro-5-(2''-chloro-4''-trifluorométhylphénoxy)-phényl]-2-thiazolin-2-yle.

15. Procédé de préparation des composés d'oxazole, d'oxazine, d'imidazole, de pyrimidine et de thiazole de formule I selon la revendication 1, caractérisé en ce qu'on traite un halogénure de l'acide 2-nitro-5-phénoxybenzoïque de formule II

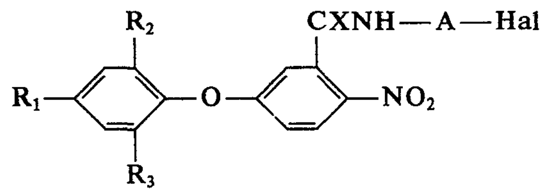

(II)

où A, $R_1$, $R_2$, $R_3$ et X ont la signification donnée dans la revendication 1, dans un solvant et diluant inertes avec un hydroxyde de métal alcalin.

16. Agent herbicide et inhibant la croissance des plantes, caractérisé en ce qu'il contient comme composant actif au moins un composé de formule I selon la revendication 1.

17. Application des composés de formule I selon la revendication 1 à la lutte contre la croissance végétale indésirable.

18. Application des composés de formule I selon la revendication 1 à la lutte sélective contre les mauvaises herbes dans les cultures de plantes utiles.

19. Application des composés de formule I selon la revendication 1 à la lutte sélective contre les mauvaises herbes dans les cultures de céréales, de riz et de maïs.

20. Application des composés de formule I selon la revendication 1 à la défoliation et à la dessication des parties aériennes des plantes.